# DEMANDE DE BREVET EUROPEEN

(11) **EP 1 201 640 A1**
(43) Date de publication de la demande: **02.05.2002**
(21) Numéro de dépôt: 01402646.2
(22) Date de dépôt: 12.10.2001
(51) Int. Cl.: C07C 67/03, C07C 69/54, C07C 69/013

(54) **Procédé de fabrication de (méth)acrylates de méthylcyclohexyle**

(30) Priorité: 25.10.2000 FR 0013672
(71) Demandeur: Atofina, 92800 Puteaux (FR)
(72) Inventeur: Paul, Jean-Michel, 57070 Metz (FR)
(74) Mandataire: Rieux, Michel

(57) **Abrégé**

On prépare un (méth)acrylate de méthylcyclohexyle de formule (I) en faisant réagir un (méth)acrylate léger (II) avec un alcool (III), en présence d'un catalyseur de transestérification choisi parmi les alcoolates de titane, d'étain, de zirconium, de magnésium, de calcium, de lithium, de potassium ou de sodium ; les chélates de zirconium, de calcium, de magnésium et de lithium avec des composés 1,3-dicarbonyle ; les dialkyloxydes d'étain, les dialkyldialcoxydes d'étain, les dialkyldiesters d'étain et les distannoxanes ; et les hydroxydes de magnésium, de calcium et de lithium. 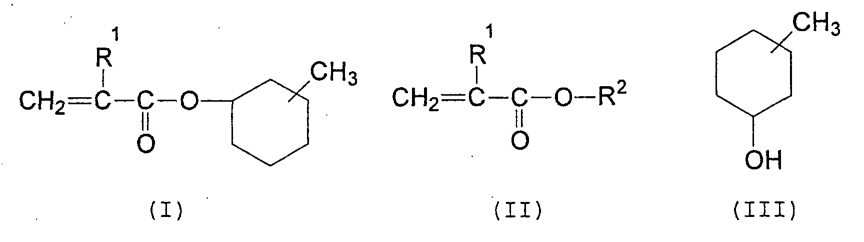 R¹ = CH₃ ; le groupe CH₃ substituant du radical cyclohexyle dans (I) et (III) pouvant occuper indifféremment les positions ortho, méta ou para ; et R² = alkyle en C₁-C₄.

## Description

La présente invention porte sur un procédé de fabrication de (méth)acrylates de méthylcyclohexyle, plus précisément des (méth)acrylates de 2-méthylcyclohexyle, de 3-méthylcyclohexyle et de 4-méthylcyclohexyle, individuellement ou en mélange d'au moins deux d'entre eux.

De tels monomères confèrent des propriétés particulières aux résines dans la composition desquelles ils entrent, en particulier par exemple une bonne résistance à la chaleur et une amélioration de certaines caractéristiques mécaniques et d'aspect des revêtements.

Différents procédés de synthèse sont décrits dans la littérature :
- estérification de l'acide acrylique par le 4-méthyl-cyclohexanol en présence d'acide para toluène sulfonique (Azerb. Khim. Zh. 1983, 3 57 - 9 ; J. Polymer Sci., 1965, 3(11)3978-81) ; et
- transestérification de l'acrylate de méthyle avec les 2-, 3- et 4-méthyl cyclohexanols en présence d'acide paratoluènesulfonique (brevets américains US-A-2 445 925 ; US-A-2 473 544).

Recherchant à obtenir ces (méth)acrylates de méthylcyclohexyle avec des rendements et sélectivités augmentés, la Société déposante a découvert une nouvelle famille de catalyseurs de transestérification permettant d'atteindre les objectifs recherchés, à savoir d'excellents rendements et une très bonne sélectivité.

La présente invention a donc pour objet un procédé de fabrication d'un (méth)acrylate de méthylcyclohexyle de formule (I): 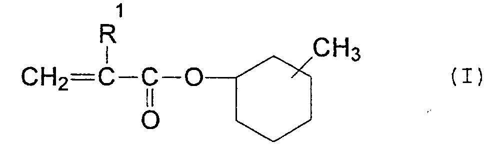 dans laquelle :
- R¹ représente H ou CH₃ ;
- le groupe CH₃ substituant du radical cyclohexyle pouvant occuper indifféremment les positions ortho, méta ou para par rapport au groupement (méth)acryloyloxy,
suivant lequel on fait réagir, en présence d'un catalyseur de transestérification, un (méth)acrylate léger de formule (II) : 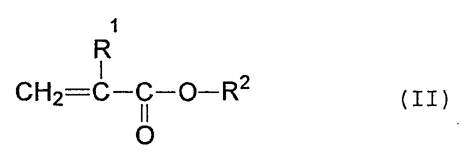 dans laquelle :
- R¹ est tel que défini ci-dessus ; et
- R² représente un radical alkyle en C₁-C₄,
avec au moins un alcool de formule (III) : 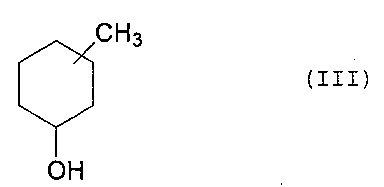 dans laquelle le groupe CH₃ substituant du radical cyclohexyle peut occuper indifféremment les positions ortho, méta ou para par rapport au groupement OH,
caractérisé par le fait que l'on utilise, comme catalyseur de transestérification, un composé choisi parmi :
- les alcoolates de titane, d'étain, de zirconium, de magnésium, de calcium, de lithium, de potassium ou de sodium ;
- les chélates de zirconium, de calcium, de magnésium et de lithium avec des composés 1,3-dicarboxyle ;
- les dialkyloxydes d'étain, les dialkyldialcoxydes d'étain, les dialkyldiesters d'étain et les distannoxanes ; et
- les hydroxydes de magnésium, de calcium et de lithium.

L'alcool (III) peut être pris sous sa forme à isomères purs cis ou trans ou de mélanges cis/trans.

On obtient des mélanges d'isomères de position du composé (I) lorsque l'on a engagé des mélanges d'isomères de position de l'alcool (III).

Des exemples d'alcoolates sont les titanates de tétraalkyle Ti(OR¹)₄ avec R¹ représentant méthyle, éthyle, butyle, isopropyle, 2-éthylhexyle ; les alcoolates de magnésium Mg(OR²)₂, R² représentant un reste alkyle en C₁-C₄, par exemple méthyle, éthyle, n-propyle, butyle.

Des exemples de chélates sont les acétylacétonates de zirconium, de calcium, de magnésium et de lithium.

Les dialkyloxydes d'étain sont notamment les composés de formule R³₂ SnO, dans laquelle R³ représente un reste alkyle en C₁-C₃₀, un exemple étant le di-n-butyloxyde d'étain Bu₂SnO (DBTO).

Les dialkyldialcoxydes d'étain sont notamment les composés de formule R⁴₂Sn(OR⁵)₂, dans laquelle R⁴ et R⁵ représentent chacun indépendamment un reste alkyle en C₁-C₃₀.

Les dialkyldiesters d'étain sont notamment les composés de formule 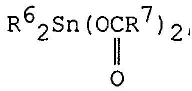 , dans laquelle R⁶ et R⁷ représentent chacun indépendamment alkyle en C₁-C₃₀, des exemples de ces composés étant le dibutyl dilaurate d'étain et le dibutyl diacétate d'étain.

Les distannoxanes sont notamment les composés de formule XR⁸₂SnOSnR⁸₂Y, dans laquelle X et Y représentent chacun indépendamment Cℓ, Br, NCS ou OH ; et les R⁸ représentent chacun alkyle en C₁-C₈, tel que méthyle ou butyle. A titre d'exemple, on peut citer le tétrabutyldichlorodistannoxane.

Conformément à la présente invention, on utilise notamment une quantité de catalyseur comprise entre 10⁻³ et 5 x 10⁻² mole, en particulier comprise entre 5 x 10⁻³ et 5 x 10⁻² mole, par mole d'alcool de formule (III).

Le(méth)acrylate léger (II) est par exemple le (méth)acrylate de méthyle, d'éthyle, de n-propyle, d'isopropyle, de n-butyle, d'isobutyle ou de tert.-butyle, étant en particulier le (méth)acrylate de méthyle.

La réaction du procédé de la présente invention peut être effectuée en présence d'un excès de l'un ou l'autre des réactifs. Il est cependant préférable d'opérer en présence d'un excès d'ester léger (II).

Ainsi, le rapport molaire ester léger (II)/alcool (III) peut être compris entre 0,7 et 7, de préférence entre 2 et 4.

Par ailleurs, on conduit la réaction du procédé de l'invention généralement en présence d'au moins un inhibiteur de polymérisation, lequel est choisi notamment parmi la phénothiazine, le butyldithiocarbamate de cuivre, l'éther monométhylique d'hydroquinone, l'hydroquinone, le ditertiobutylparacrésol, le 2,2,6,6-tétraméthyl-1-pipéridinyloxy (TEMPO), le 4-hydroxy-2,2,6,6-tétraméthyl-1-pipéridinyloxy (4-hydroxy-TEMPO), le 4-méthoxy-2,2,6,6-tétraméthyl-1-pipéridinyloxy (4-méthoxy-TEMPO), le 4-oxo-2,2,6,6-tétraméthyl-1-pipéridinyloxy (4-oxo-TEMPO), et leurs mélanges en toutes proportions.

On introduit le ou les inhibiteurs de polymérisation à raison notamment de 0,05 à 0,5 % en masse par rapport à l'alcool de formule (III).

On conduit la réaction du procédé de l'invention de préférence sous pression réduite afin de maintenir la température du mélange réactionnel en dessous de 120°C.

La durée de la réaction est fonction des conditions opératoires mais est généralement comprise entre 5 et 8 heures.

Conformément à un mode de réalisation particulier du procédé selon la présente invention, on conduit la réaction selon les étapes successives suivantes :
- mélange du (méth)acrylate léger de formule (II), du ou des alcools (III) et, le cas échéant, du ou des inhibiteurs de polymérisation, et chauffage à reflux afin d'éliminer les traces d'humidité résiduelle sous forme d'un azéotrope (méth)acrylate léger (II)/eau ;
- lorsque l'étape de séchage est terminée, introduction du catalyseur et démarrage de la phase réactionnelle, l'alcool léger qui se forme lors de la réaction étant éliminé sous la forme d'un azéotrope (méth)acrylate léger (II)/alcool léger ;
- purification par distillation du brut réactionnel par élimination de l'ester léger résiduel (II) et de l'alcool résiduel (III) sous la forme de deux fractions de distillation ; et récupération du composé (I) recherché ou de mélange de composés (I) recherchés sous forme d'une troisième fraction de distillation.

Les exemples suivants illustrent la présente invention sans toutefois en limiter la portée. Dans ces exemples, les pourcentages sont des pourcentages massiques sauf indication contraire et les abréviations suivantes ont été utilisées :
MAM : méthacrylate de méthyle
Zr(acac)₂: acétylacétonate de zirconium
DBTO : di-n-butyloxyde d'étain Bu₂SnO

### EXEMPLE 1 :

Dans un réacteur agité à l'aide d'un agitateur type ancre de 1 litre en verre, chauffé à l'aide d'une double enveloppe alimentée par de l'huile thermostatée, surmonté d'une colonne à distiller à garnissage d'efficacité égale à 4 plateaux, on introduit successivement :
- 251,2 g de 3-méthylcyclohexanol ;
- 660 g de MAM ;
- 0,80 g de phénothiazine ; et
- 0,80 g de dibutyl dithiocarbamate de cuivre.

On effectue successivement les diverses opérations suivantes :

### • Séchage :

On chauffe à reflux, à la pression atmosphérique en distillant une tête contenant 0,05 % d'eau (fraction F0 : 50 g).

Pendant toute la durée de l'essai on maintient un bullage d'air dans le mélange réactionnel.

### • Réaction :

Lorsque le séchage est terminé on introduit le catalyseur (8,22 g, soit 0,015 mole/mole de 3-méthyl-cyclohexanol) de DBTO. La pression est réduite progressivement jusqu'à 6,66 x 10⁴ Pa (500 mmHg), afin de maintenir la température dans le réacteur en dessous de 100°C.

On récupère en tête de colonne 125 g d'un mélange constitué par 56,9 % de méthanol et 42,7 % de méthacrylate de méthyle (fraction F1).

La durée de réaction est de 6,5 h.

Le taux de conversion calculé d'après le méthanol formé est > 99 %.

### • Distillation :

L'excès de MAM et les traces d'alcool résiduel (III) sont éliminés par distillation en baissant progressivement la pression de 6,66 x 10⁴ Pa (500 mmHg) à 3,99 x 10³ Pa (30 mmHg) afin de maintenir la température dans le réacteur à 115°C maximum (fraction F2 : 365 g).

Le méthacrylate de 3-méthylcyclohexyle est distillé sous 2,66 x 10³ Pa (20 mmHg) (fraction F3 : 348 g).

Le méthacrylate de 3-méthylcyclohexyle obtenu en final est un mélange d'isomères cis-trans. Il a été caractérisé par RMN.

### EXEMPLES 2 à 7 :

Dans les mêmes conditions générales qu'à l'Exemple 1, mais en faisant varier le rapport molaire MAM/alcool (III), la nature de l'alcool (III), la nature du catalyseur et le taux de ce dernier, on a préparé six composés ou mélanges de composés de l'invention.

Les résultats sont rapportés dans le Tableau 1.

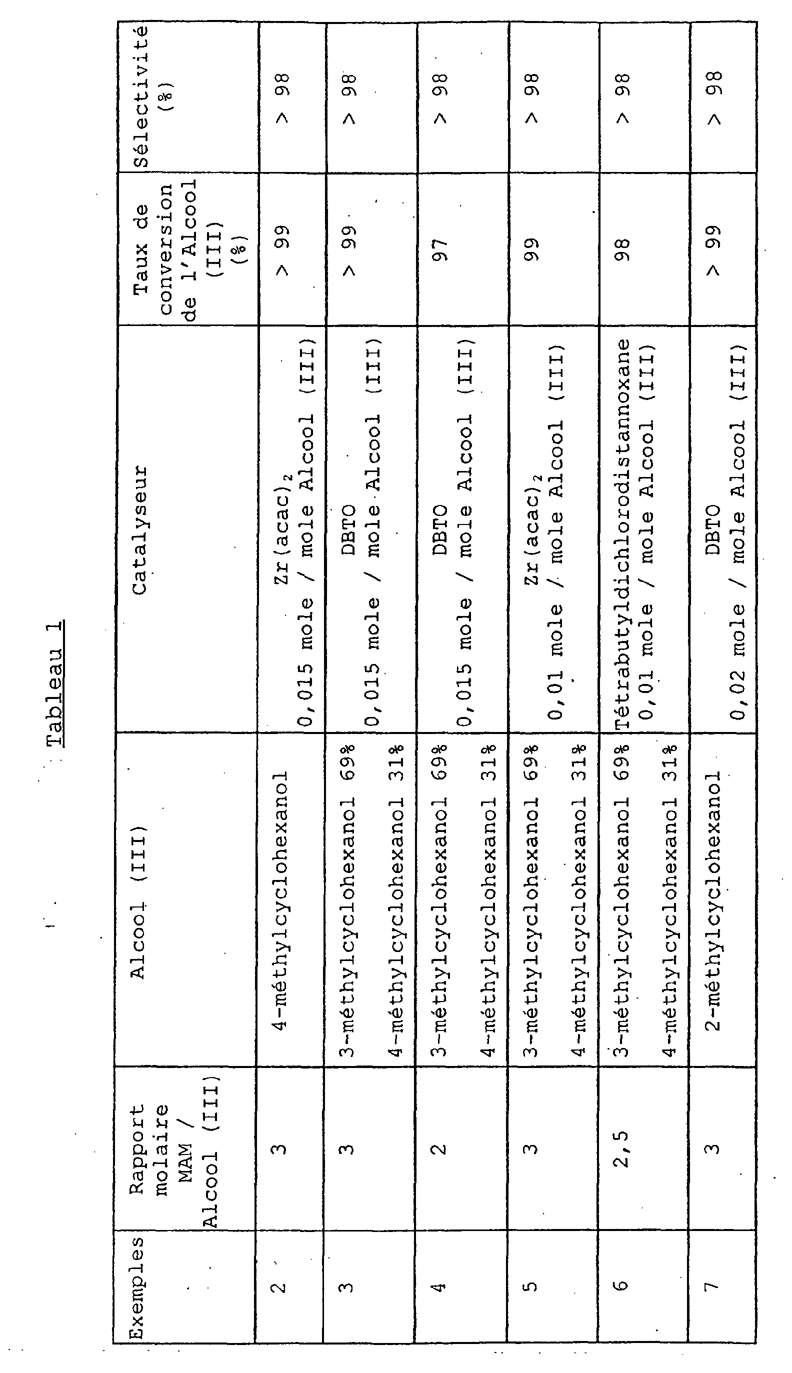

## Revendications

1. Procédé de fabrication d'un (méth)acrylate de méthylcyclohexyle de formule (I) : 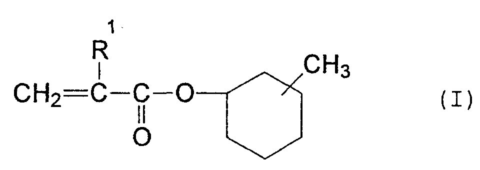 dans laquelle :
- R¹ représente H ou CH₃ ;
- le groupe CH₃ substituant du radical cyclohexyle pouvant occuper indifféremment les positions ortho, méta ou para par rapport au groupement (méth)acryloyloxy,
suivant lequel on fait réagir, en présence d'un catalyseur de transestérification, un (méth)acrylate léger de formule (II) : 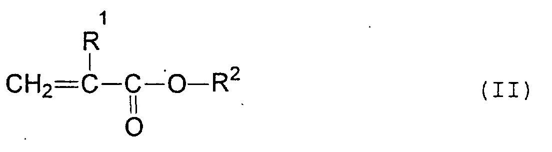 dans laquelle :
- R¹ est tel que défini ci-dessus ; et
- R² représente un radical alkyle en C₁-C₄,
avec au moins un alcool de formule (III) : 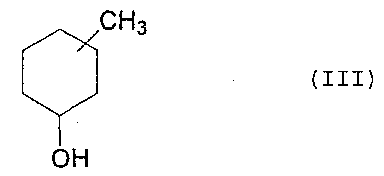 dans laquelle le groupe CH₃ substituant du radical cyclohexyle peut occuper indifféremment les positions ortho, méta ou para par rapport au groupement OH,
**caractérisé par le fait que** l'on utilise, comme catalyseur de transestérification, un composé choisi parmi les alcoolates de titane, d'étain, de zirconium, de magnésium, de calcium, de lithium, de potassium ou de sodium ; les chélates de zirconium, de calcium, de magnésium et de lithium avec des composés 1,3-dicarbonyle ; les dialkyloxydes d'étain, les dialkyldialcoxydes d'étain, les dialkyldiesters d'étain et les distannoxanes ; et les hydroxydes de magnésium, de calcium et de lithium.

2. Procédé selon la revendication 1, **caractérisé par le fait que** l'on utilise, comme catalyseur, un composé choisi parmi les titanates de tétraalkyle Ti(OR¹)₄ avec R¹ représentant méthyle, éthyle, butyle, isopropyle, 2-éthyl-hexyle ; les alcoolates de magnésium Mg(OR²)₂, R² représentant un reste alkyle en C₁-C₄, par exemple méthyle, éthyle, n-propyle, butyle ; les acétylacétonates de zirconium, de calcium, de magnésium et de lithium ; le di-n-butyloxyde d'étain, le dibutyldilaurate d'étain ; le dibutylacétate d'étain ; et le tétrabutyldichlorodistannoxane.

3. Procédé selon l'une des revendications 1 et 2, **caractérisé par le fait que** l'on utilise une quantité de catalyseur comprise entre 10⁻³ et 5 x 10⁻² mole par mole d'alcool de formule (III).

4. Procédé selon la revendication 3, **caractérisé par le fait que** l'on utilise une quantité de catalyseur comprise entre 5 x 10⁻³ et 5 x 10⁻² mole par mole d'alcool de formule (III).

5. Procédé selon l'une des revendications 1 à 4, **caractérisé par le fait que** l'on utilise, comme (méth)acrylate léger (II), le (méth)acrylate de méthyle, d'éthyle, de n-propyle, d'isopropyle, de n-butyle, d'isobutyle ou de tert.-butyle.

6. Procédé selon l'une des revendications 1 à 5, **caractérisé par le fait que** l'on effectue la réaction en présence d'un excès de l'un ou l'autre des réactifs.

7. Procédé selon la revendication 6, **caractérisé par le fait que** l'on conduit la réaction avec un rapport molaire ester léger (II)/alcool (III) compris entre 0,7 et 7.

8. Procédé selon la revendication 7, **caractérisé par le fait que** l'on conduit la réaction avec un rapport molaire ester léger (II)/alcool (III) compris entre 2 et 4.

9. Procédé selon l'une des revendications 1 à 8, **caractérisé par le fait que** l'on conduit la réaction en présence d'au moins un inhibiteur de polymérisation choisi notamment parmi la phénothiazine, le butyldithiocarbamate de cuivre, l'éther monométhylique d'hydroquinone, l'hydroquinone, le ditertiobutylparacrésol, le 2,2,6,6-tétraméthyl-1-pipéridinyloxy (TEMPO), le 4-hydroxy-2,2,6,6-tétraméthyl-1-pipéridinyloxy (4-hydroxy-TEMPO), le 4-méthoxy-2,2,6,6-tétraméthyl-1-pipéridinyloxy (4-méthoxy-TEMPO), le 4-oxo-2,2,6,6-tétraméthyl-1-pipéridinyloxy (4-oxo-TEMPO).

10. Procédé selon la revendication 9, **caractérisé par le fait que** l'on introduit le ou les inhibiteurs de polymérisation à raison de 0,05 à 0,5 % en masse par rapport à l'alcool de formule (III).

11. Procédé selon l'une des revendications 1 à 10, **caractérisé par le fait que** l'on conduit la réaction sous pression réduite afin de maintenir la température du mélange réactionnel en dessous de 120°C.

12. Procédé selon l'une des revendications 1 à 11, **caractérisé par le fait que** l'on conduit la réaction pendant un laps de temps compris entre 5 et 8 heures.

13. Procédé selon l'une des revendications 1 à 12, **caractérisé par le fait que** l'on conduit la réaction selon les étapes successives suivantes :
- mélange du (méth)acrylate léger de formule (II), du ou des alcools (III) et, le cas échéant, du ou des inhibiteurs de polymérisation, et chauffage à reflux afin d'éliminer les traces d'humidité résiduelle sous forme d'un azéotrope (méth)acrylate léger (II)/eau ;
- lorsque l'étape de séchage est terminée, introduction du catalyseur et démarrage de la phase réactionnelle, l'alcool léger qui se forme lors de la réaction étant éliminé sous la forme d'un azéotrope (méth)acrylate léger (II)/alcool léger ;
- purification par distillation du brut réactionnel par élimination de l'ester léger résiduel (II) et de l'alcool résiduel (III) sous la forme de deux fractions de distillation ; et récupération du composé (I) recherché ou de mélange de composés (I) recherchés sous forme d'une troisième fraction de distillation.
